Europäisches Patentamt

European Patent Office

Office européen des brevets

(11) Publication number: **0 323 802**
**A2**

# EUROPEAN PATENT APPLICATION

(21) Application number: **88810885.9**

(22) Date of filing: **21.12.88**

(51) Int. Cl.4: **C12P 21/00 , A61K 39/00 ,
C12N 15/00**

The applicant has filed a statement in accordance with Rule 28 (4) EPC (issue of a sample only to an expert). Accession number(s) of the deposit(s): ECACC 88030201.

Claims for the following Contracting States: ES + GR.

(30) Priority: **24.12.87 GB 8730185**
**01.06.88 GB 8812963**
**24.08.88 GB 8820035**

(43) Date of publication of application:
**12.07.89 Bulletin 89/28**

(84) Designated Contracting States:
**AT BE CH DE ES FR GB GR IT LI LU NL SE**

(71) Applicant: **SANDOZ AG**
**Lichtstrasse 35**
**CH-4002 Basel(CH)**

(84) **BE CH ES FR GB GR IT LI LU NL SE**

Applicant: **SANDOZ-PATENT-GMBH**
**Humboldtstrasse 3**
**D-7850 Lörrach(DE)**

(84) **DE**

Applicant: **SANDOZ-ERFINDUNGEN**
**Verwaltungsgesellschaft m.b.H.**
**Brunner Strasse 59**
**A-1235 Wien(AT)**

(84) **AT**

(72) Inventor: **Stahel, Rolf A.**
**Höhenstrasse 48**
**CH-8127 Forch(CH)**
Inventor: **Waibel, Robert**
**Räterschenstrasse 5**
**CH-8352 Ricketwil(CH)**

(54) **Monoclonal antibodies against, and cell surface antigen of, lung carcinoma.**

(57) Monoclonal antibodies and fragments thereof, which identify a specific cell surface membrane expressed sialoglycoprotein antigen of the small cell carcinoma of the lung, hybrid cell lines producing these antibodies, methods of using the antibodies and the specific antigen itself, named $sGP_{100}$, are disclosed. A preferred antibody is SWA20. The antibodies are indicated for use in immunoassays and for immunotherapy of small cell carcinoma of the lung.

EP 0 323 802 A2

## MONOCLONAL ANTIBODIES AGAINST, AND CELL SURFACE ANTIGEN OF, LUNG CARCINOMA

### FIELD

This invention is directed to monoclonal antibodies against a cell surface antigen of the small cell carcinoma of the lung, hybridoma cell lines producing these antibodies, methods of using these monoclonal antibodies and the cell surface antigen itself.

### BACKGROUND ART

A variety of investigations have been performed in an attempt to determine the characteristic properties of the small cell carcinoma of the lung. Included among these have been investigations into the in vitro growth morphology (Cancer Res. 40 3500 [1980]), the expression of enzymes such as dopamine carboxylase (Cancer Res. 40 1990 [1980]), the expression of the polypeptide hormone bombesin (Science 214 1246 [1981]) and deletions on chromosome 3p (Science 115 181 [1982]). It has been shown that small cell carcinoma vary in their expression of cell surface proteins from non-small cell carcinoma of the lung (PNAS 79 4650 [1982]) and since then several investigations have led to the production of monoclonal antibodies against cell surface antigens of the small cell carcinoma of the lung.

These cell surface antigens may be classified according to tissue reactivity, such groups being epithelial antigens, neuroendocrine antigens, antigens which are shared with white blood cells and the tumour-associated antigens. Numerous antibodies have been described which have been found to react for example, against structures expressed on small cell carcinoma but also against structures expressed on normal epithelium and other carcinomas. Similarly there have been described antibodies against the neuroendocrine type antigens which recognise antigens on both normal and malignant neuroendocrine tissues, peripheral nerve and brain.

By identifying and targeting specific antigens of the small cell carcinoma of the lung using monoclonal antibodies, some membrane antigens have been isolated and characterised, and it has been shown that many of these are glycolipids. Characterisation by immunoprecipitations has suggested some other small cell carcinoma antigens to be proteins, for example character isation by immunoblotting procedures have been reported on the small cell carcinoma antigen MOC-1 (Cancer Res. 45 2192 [1985]). In similar immunoprecipitation studies an antibody was described which appeared to recognise a glycoprotein and further investigations have shown this to be a sialylated glycoprotein located in the cell surface membrane of a proportion of small cell carcinoma cell lines and tissues (Cancer Res. 47 3766 [1987]). The antigen was designated $sGP_{90-135}$ and a monoclonal antibody which identifies it as LAM8. Further characterisation of the antibody, and others showing a similar specificity, for example SWA4 and SWA23, has shown these to be of IgM isotype.

The discovery that the cell surface membrane of small cell carcinoma cells contains sialoglycoproteins and the ability to identify such antigens using monoclonal antibodies suggests the clinical usefulness of these antigens in detecting and diagnosing, and possibly in the immunotherapy, of small cell carcinoma of the lung. It has been proposed that the biological behaviour of tumour cells might be related to changes in the sialylation of membrane glycoproteins. For example an increased membrane content of sialic acid was found to be associated with decreased transplantability of human and mouse myelomas (Int. J. Cancer 36 461 [1986]), increased metastatic potential of murine tumour cells (Science 212 1514 [1981]) and resistance to natural killer cell- mediated cytotoxicity (Cancer Res. 46 4543 [1986]).

### SUMMARY

The present invention focusses on the description of surface membrane characteristics of the small cell carcinoma.

It is an object of the present invention to provide monoclonal antibodies, particularly monoclonal antibodies of IgG isotype, which are capable of reacting selectively with a specific cell surface membrane-expressed sialoglycoprotein antigen of the small cell carcinoma of the lung.

It is another object of the present invention to provide a specific sialylated glycoprotein antigen of the small cell carcinoma of the lung which may be used in the diagnosis of small cell carcinoma of the lung.

It is a further object of the present invention to provide methods for the diagnosis of small cell carcinoma of the lung using monoclonal antibodies which react selectively with a specific cell surface membrane- expressed sialoglycoprotein antigen of the small cell carcinoma of the lung.

Still another object of the present invention is to provide methods for suppressing small cell carcinoma of the lung in an animal using monoclonal antibodies which react selectively with a specific cell surface membrane-expressed sialoglycoprotein antigen of the small cell carcinoma of the lung.

The present invention thus relates to new monoclonal antibodies which are specific for a new tumour-associated sialoglycoprotein antigen strongly expressed on a proportion of small cell carcinoma, but which are non-reactive with non-small cell carcinoma. The invention further includes hybridoma cell lines which produce these antibodies as well as methods for using and processes for preparing these monoclonal antibodies.

The ability of the monoclonal antibodies to react with small cell carcinoma of the lung whilst remaining essentially non-reactive with non-small cell carcinoma and normal tissues is very significant in terms of the detection of these antigens and the immunotherapeutic use of these monoclonal antibodies.

## DESCRIPTION OF THE FIGURES

**Figure 1:** graph showing the distribution amongst tissues of the monoclonal antibody SWA20 four days after administration, in comparison with normal IgG (NIgG). Tu = tumor (1.1); Li = liver (0.27); Ki = kidney (0.37); Sp = spleen (0.38); He = heart (0.42); Th = thyroid (0.39); Lu = lung (0.43); Mu = muscle (0.15); Br = brain (0.038).

**Figure 2:** immunoblot of SW2 cells showing the effect of various enzymes on antigens $sGP_{90-135}$ and $sGP_{100}$ prior to development with antibodies SWA20 (A) and, respectively, LAM8 (B). The lanes correspond to treatment with the following compounds:
1, $Le^a$ positive saliva; 2, trypsin; 3, chymotrypsin; 4, neuraminidase; 5, mixed glycosidases; 6, periodate; 7, untreated control.
Molecular weight markers (M) in thousands are:
$\alpha_2$-macroglobulin (180,000); $\beta$-galactosidase (116,000); fructose-6-phosphate kinase (84,000); pyruvate kinase (58,000); fumarase (48,500); lactic dehydrogenase (36,500); and triosephosphate isomerase (26,600).
>: individual lane markers.

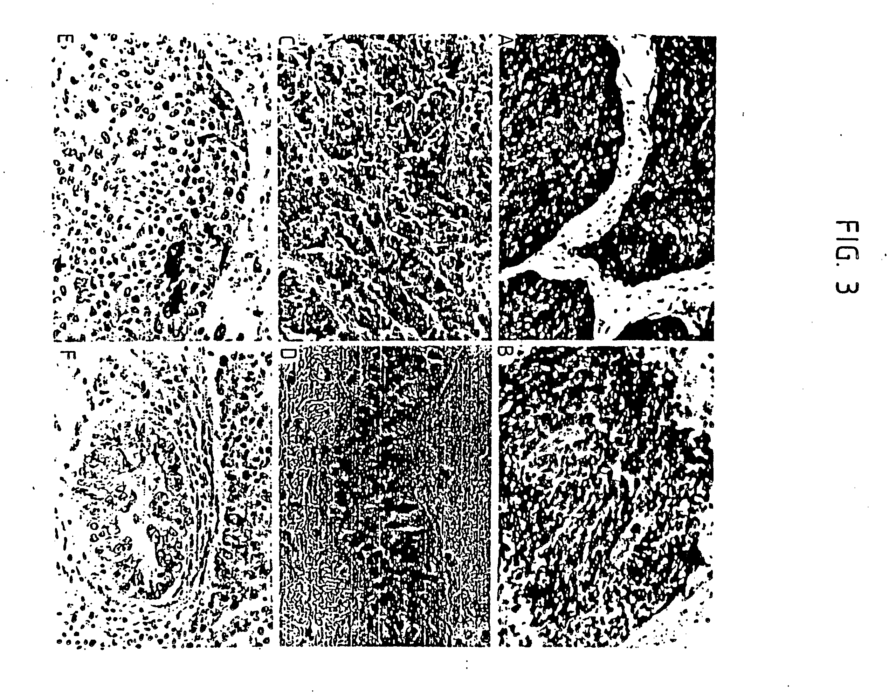

**Figure 3:** immunoperoxidase staining of paraffin sections with antibody SWA20. A) SCC with homogeneous staining, B) SCC with 50% positivity, C) SCC with less than 10% scattered positive cells, D) normal bronchus with rare positive cells, E) lung squamous cell carcinoma with less than 10% positive cells and F) lung adenocarcinoma with rare positive cells.

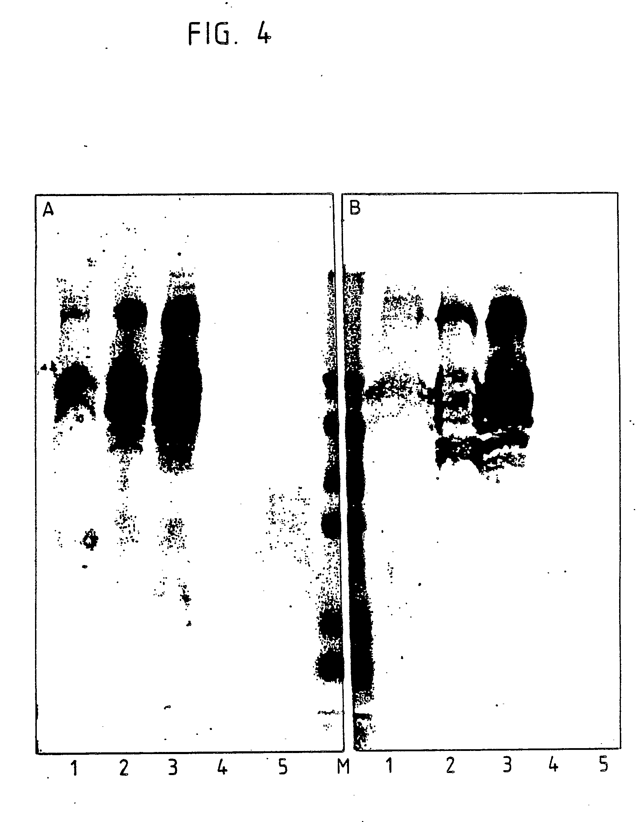

**Figure 4:** immunoblot of small cell carcinoma cell line extracts developed with antibodies SWA20 (A) and LAM8 (B). (M) molecular weight markers. Lanes contain GEM95 (lane 1); NCI-H69 (lane 2); SW2 (lane 3); OH3 (lane 4) and NCI-H128 (lane 5).

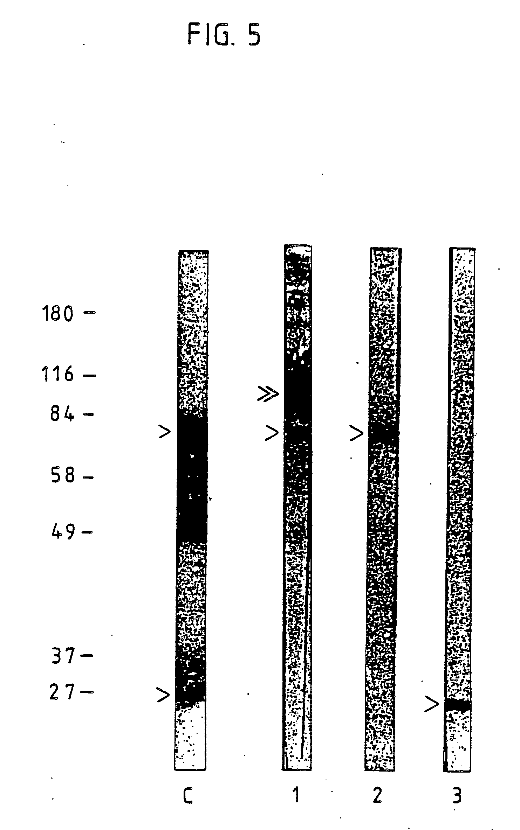

**Figure 5:** immunoblot of immunoadsorbed LAM8 antigen developed with different antibodies and stained with goat anti-mouse IgM(μ) and goat anti-mouse IgG.
Lanes contain Coomassie blue staining of cell extract immunoadsorbed with LAM8 (lane C); immunoadsorbed extract developed with LAM8, stained with anti-IgM (lane 1); immunoadsorbed extract developed with SWA4, stained with anti-IgM (lane 2); and immunoadsorbed extract developed with SWA20 and stained with anti-IgG (lane 3).
>: IgG and IgM heavy and light chains;
> >: antigen $sGP_{90-135}$.

## DETAILED DESCRIPTION

This invention relates to monoclonal antibodies of a defined isotype which identify a novel antigen selectively expressed on the cell surface membrane of a proportion of cells of the small cell carcinoma of the lung. Detection of this antigen, both in vivo and in vitro has potential for both the diagnosis and possible immunotherapy of the small cell carcinoma of the lung.

A variety of cell surface antigens of the small cell carcinoma of the lung have been characterised, such including the epitheleal antigens, neuroendocrine antigens and the antigens which are shared with white blood cells. The antigen of the present invention however, has been demonstrated to be a tumour-associated antigen which is selectively expressed on the small cell carcinoma of the lung and which shows essentially no expression on other pulmonary and non-pulmonary cancers and normal tissue.

The precise distribution of the antigen with respect to various tissues, and cell lines, has been demonstrated using monoclonal antibodies which specifically identify and bind to the antigen. The general methods for the production of a hybridoma which secretes such monoclonal antibodies is well known to those of ordinary skill in the art. Illustrative of the techniques employed are those described in Cancer Res. 46 (1986) 2077. Briefly BALB-C mice were immunised with viable cells of the SW2 or OH-1 small cell carcinoma cell line. After sacrifice of the animals, the spleen cells were fused with the NS-1 myeloma cell line and the hybridomas were screened for their ability to produce antibodies using the SW2 or OH-1 cell line. Further screening, including showing negative reactivity with bone marrow and screening for IgG isotype led to the selection of hybridoma cell lines secreting specific monoclonal antibodies. Examples of such cell lines are the lines which are designated SWA20, SEN3, SEN11, SEN12 and SEN31. The isotype of e.g. antibody SWA20 was shown to be IgG2a by double radial diffusion of antibody containing ascites against rabbit anti-mouse immunoglobulins and by immunoblotting techniques (Cancer Res. 46 [1986] 2077) using a panel of different IgG antibodies.

The hybridoma cell line designated SWA20 producing this antibody (SWA20) was deposited at the European Collection of Animal Cell Cultures (ECACC) on 2nd March 1988 and received the deposit number ECACC 88030201.

It is also foreseen that anti-idiotype antibodies may be produced, such techniques being within the capabilities of a person of ordinary skill in the art. Briefly, an anti-idiotype antibody is an antibody which recognises unique determinants present on the antibody of interest. Thus a monoclonal antibody such as SWA20 is injected into an animal such as was used as the source of the original monoclonal antibody-producing cell. The animal immunised will recognise and respond to the idiotypic determinants of the immunising antibody by producing an antibody to these idiotypic determinants (the anti-idiotypic antibody). These anti-idiotypic antibodies, which are specific for a monoclonal antibody itself specific for the particular antigen of interest, will, if injected into a further host, stimulate the production of anti-(anti-idiotypic) antibodies. These anti-(anti-idiotypic) antibodies will be of the same specificty as the original monoclonal antibody and will themselves identify the antigen of interest. The advantage of employing such a system is that the antibodies produced in the final host which bears the antigen of interest, are endogenous to that host and may be produced naturally in vast quantities. Also, only very small amounts of anti-idiotype antibody need be administered to the final host, such amounts being sufficient only to stimulate an immune response.

Anti-idiotype antibodies may also be used in the isolation of other hybridoma cell lines secreting monoclonal antibodies having the specificity of the monoclonal antibodies of the invention. Such antiidiotype screening may readily be accomplished by one of ordinary skill in the art. Briefly, anti-idiotype antibodies are produced as described before, and by using these anti-idiotype antibodies, which are specific for the monoclonal antibodies produced by a single hybridoma, it is possible to identify other clones with exactly the same idiotype as the antibody of the hybridoma used for immunisation. Idiotypic identity between monoclonal antibodies of two hybridomas demonstrates that the two monoclonal antibodies are the same with respect to their recognition of the same epitopic determinant. Thus, by using antibodies to the epitopic determinants on a monoclonal antibody it is possible to identify other hybridomas expressing monoclonal antibodies of the same epitopic specificity. In antibodies, these idiotypic determinants are present in the hypervariable region which binds to a given epitope.

While the use of a foreign donor monoclonal antibody of one species in a second recipient species is usually not a factor in in vivo immunotherapy or immunodiagnosis, a potential problem which may arise is the occurrence of an adverse immunological response by the host to antigenic determinants present on the donor antibody. In some instances, this adverse response can be so severe as to curtail the in vivo use of the donor antibody in the host. Further, this adverse host response may serve to hinder the malignancy-suppressing efficacy of the donor antibody. One way in which it is possible to circumvent the likelihood of an adverse immune response occuring in the host is by using chimeric antibodies (Sun et al., Hybridoma 5 (1) S17 [1986] and Oi et al., Bio Techniques 4 (3) 214 [1986]). Chimeric antibodies are antibodies in which the various domains of the antibody heavy and light chains are coded for by DNA from more than one species. Typically, a chimeric antibody will comprise the variable domains of the heavy ($V_H$) and light ($V_L$) chains derived from the donor species producing the antibody of desirable antigenic specificity and the constant antibody domains of the heavy ($C_H$) and light ($C_L$) chains derived from the host recipient species. It is believed that by reducing the exposure of the host immune system to the antigenic determinants on the donor antibody domains the possibility of an adverse immunological response occuring in the recipient species will be reduced. Thus, for example, it is possible to produce a chimeric antibody for in vivo clinical use in humans which comprises mouse $V_H$ and $V_L$ domains coded for by DNA isolated from the SWA20 cell line deposited under ECACC 88030201 and $C_H$ and $C_L$ domains coded for by DNA isolated from a human cell.

Under certain circumstances, monoclonal antibodies of a particular class may be preferable, in terms of diagnostic or therapeutic efficacy, over those of a different class. For example problems may be associated with the utilisation of antibodies of the IgM class, such including high cross-reactivity in tissue staining, poor tissue penetration and difficulties with quantitative biochemical manipulations such as the production of immunoconjugates. Similarly monoclonal antibodies of a particular isotype might be preferable to those of another. In this respect it is known that mouse monoclonal antibodies of isotype gamma-2a and gamma-3 are more effective in inhibiting the growth of tumours than is the gamma-1 isotype. This differential efficacy is thought to be due to the ability of the gamma-2a and gamma-3 isotypes to participate more actively in cytolytic destruction of tumour cells. Particular isotypes of a monoclonal antibody can be prepared either directly by selecting from the initial hybridoma fusion or prepared secondarily from a parental hybridoma secreting a monoclonal antibody of different isotype and selecting for class-switch variants having the same specificity as, for example, SWA20.

The monoclonal antibodies of the invention including chimeric or antiidiotypic antibodies having the same specificity or stimulating the production of antibodies with the same specificity respectively, can be used in any animal in which it is desirable to administer in vitro or in vivo immunodiagnosis or immunotherapy. The term "animal" as used herein is meant to include both humans as well as non-humans.

The term "antibody" as used in this invention is meant to include intact molecule as well as fragments thereof, such as, for example, Fab and F(ab')$_2$, which are capable of binding the epitopic determinant.

The term "essentially non-reactive" when used to characterise the reactivity between the monoclonal antibodies of the invention and an antigen means that any reaction which might occur is insignificant in terms of limiting the diagnostic or therapeutic usefulness of the antibodies.

The monoclonal antibodies of the invention are particularly suited for use in immunoassays in which they can be used in liquid phase or bound to a solid phase carrier. In addition, the monoclonal antibodies in these immunoassays can be detectably labeled in various ways. Examples of types of immunoassay which can use the monoclonal antibodies of the invention are competitive and non-competitive immunoassays, common examples being the radioimmunoassay (RIA) and the enzyme-linked immunosorbent assay (ELISA). There are many different labels and methods of labeling for the monoclonal antibodies, and those of ordinary skill in the art will either know of such or will be able to ascertain such using routine experimentation and will be able to effect binding using standard techniques.

Employing such techniques the monoclonal antibodies of the invention have been used to determine the distribution of the antigen of the invention with respect to cell lines and tissues of both small cell carcinoma and non-small cell carcinoma origin, and with respect to the location of the antigen in the cell of the small cell carcinoma of the lung. Investigations into the expression of the small cell carcinoma sialoglycoprotein antigen $SGP_{90-135}$ (Cancer Res. 47 [1987] 3766) and the discovery that this antigen is strongly expressed on a proportion of small cell carcinoma of the lung, have allowed this antigen to be used as a control in the determination of the expression of the antigen of the present invention, $sGP_{100}$.

The fact that the antigen $sGP_{100}$ is expressed in the cell surface membrane of small cell carcinoma was demonstrated using indirect immunofluorescence staining, the methods employed being such as those described in Cancer Res. 46 (1986) 2077 and Int. J. Cancer 35 (1985) 11. Using such techniques the binding of the monoclonal antibody SWA20 to the small cell carcinoma cell line SW2 or OH-1 produces a ring type staining, such being indicative of cell surface membrane activity. Similar results are obtained when other small cell carcinoma cell lines are used, but no positive result is obtained with cell lines of non-small cell carcinoma origin.

Solid phase radioimmunoassays (RIA) were performed on a variety of cell lines both of small cell carcinoma and non-small cell carcinoma origin using the monoclonal antibodies of the invention (the methods being employed being such as those described in Example 3), and these demonstrated that $sGP_{100}$ is strongly expressed on four out of the eight small cell carcinoma lines employed (Figure 4), but shows no expression on non-small cell carcinoma cell lines. Non-small cell carcinoma cell lines used include breast carcinoma, colon carcinoma and leukaemia and on-cancerous cells tested include normal bronchial epithelial cells from primary cultures, white blood cells from peripheral blood (buffy coat) and mono-nuclear cells from bone marrow. The antibody was also unreactive with red blood cells expressing the major blood group antigens in haemagglutination and complement lysis assays.

The antigen of the invention, $sGP_{100}$, is not only specific to small carcinoma cell lines but the specificity of its expression has also been demonstrated in various tissue samples using immunoperoxidase staining. Initial experiments with such staining of small cell carcinoma tissues demonstrated preservation of the antigen in formalin-fixed, paraffin-embedded tissues. The methods used in the staining technique are exemplified in Example 4. Of a series of lung tumour and extra-pulmonary tumour tissues stained with the antibody SWA20, positive staining was restricted to small cell carcinoma (Figure 3). Less than 10% positivity of cells scattered throughout the tumour tissue samples from adenocarcinoma, squamous cell carcinoma and breast carcinoma showed positive staining, but all other samples were clear of staining. Out of the 12 different small cell carcinomas examined, 2 cases showed homogeneous staining of virtually all cells, 3 cases showed approximately 50% positivity and 2 cases about 25% positivity. Three cases had less than 10% focal positivity and 2 were entirely negative.

Normal, non-cancerous tissues were also included in this investigation, for example kidney, spleen, anterior pituitary and lung parenchyma. Out of the 16 types of tissue examined, all were entirely negative apart from rare positive cells in lung bronchus and pancreas islets and less than 10% positivity in colon epithelial cells.

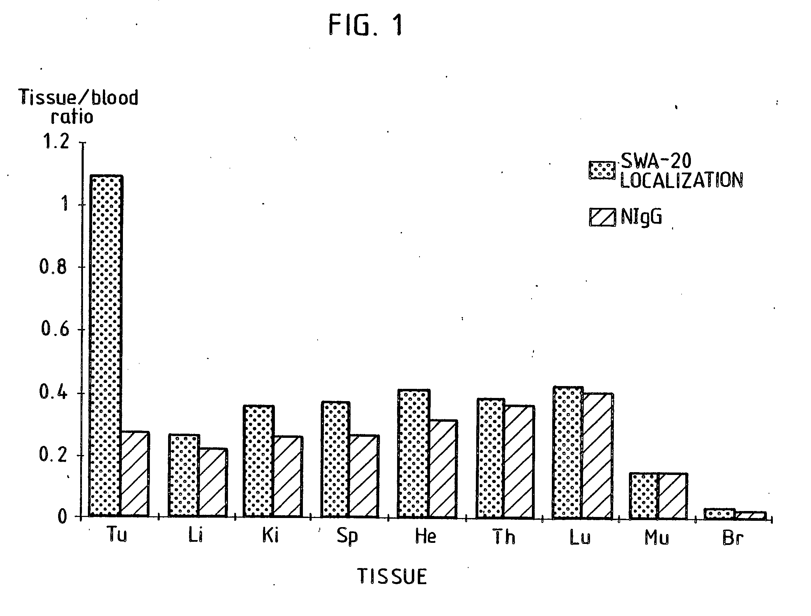

In an in vivo analysis of the uptake of one monoclonal antibody of the invention, the antibody SWA20 was labeled with $^{125}I$ by the iodogen technique and was found to retain a biological activity of around 46%. When injected intravenously into nude mice bearing SW2 or OH-1 small cell carcinoma xenografts, SWA20 was found to localise preferentially to the heterotransplants, achieving a level four-fold higher than a control immunoglobulin. The absolute value of localising SWA20 was 7% of injected dose/gm of tumour (i.e. 15% in terms of biologically active material). Thus, selective antibody uptake in small cell carcinoma heterotransplants in nude mice was demonstrated. Figure 1 shows the localisation of labeled monoclonal antibody SWA20 in various tissues after such an administration, in comparison with the localisation of normal labeled IgG. It may readily be seen that the largest percentage of the SWA20 is to be found in the tumour tissue.

The indirect immunofluorescence staining indicated that $sGP_{100}$ is located in the cell surface membrane of cells of small cell carcinoma of the lung and experiments were performed to determine with which fraction of the membrane the antigen is associated. Upon separation of the components of the membrane into its various fractions, no antigen was observed in the crude glycolipid extracts using thin layer chromatography on silica gels. Electrophoresis of small cell carcinoma cells on 10% SDS-polyacrylamide gels under reducing conditions using the buffer system of O'Farrel, followed by transfer of proteins, electrophoretically, onto 0.2 μm nitrocellulose according to the method of Otter (Anal. Biochem. 162 [1987] 370) in a two-step process beginning with the elution of low molecular weight molecules at low current density followed by prolonged electrotransfer at high current density, and incubation with the antibody supernatant and affinity-purified peroxidase-conjugated goat anti-mouse IgM or IgG, allowed for the detection of antigen in the protein component of the small cell carcinoma cell membrane. The presence of antigen was demonstrated by the measurement of peroxidase activity using 4-chloro-1-naphthol as substrate. The antigen $sGP_{100}$ has been demonstrated, by biochemical characterisation, to be a sialylated glycoprotein. Such techniques include treating the isolated antigen with the enzyme neuraminidase, which cleaves sialic acid residues from a carbohydrate moiety, and determining whether the antibody remains reactive with an antigen so treated.

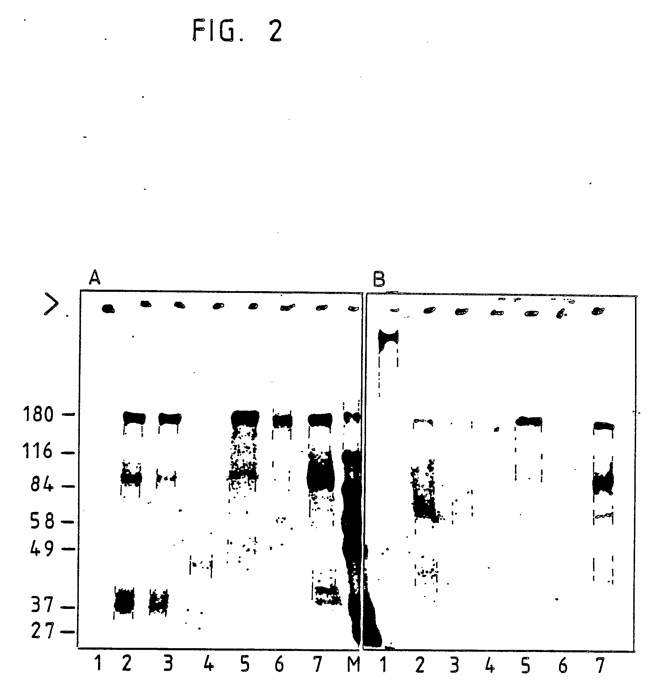

As illustrated in Figure 2 the antigen $sGP_{100}$, under conventional immunoblotting techniques using the monoclonal antibody SWA20, produces several molecular weight bands at MW 40,000, a broader band at MW 100,000 and a band at MW 180,000 with the 50-75 % ammonium sulphate fraction.

To demonstrate the nature of the antigen, competition radioimmunoassays were performed on the SW2 cell line using antibody supernatant and the lectins Ulex europaeus, Triticum vulgaris and Concanavalin A. Lectins are proteins, usually isolated from plant material, which bind to specific sugar moieties, for example the lectin Ulex europaeus recognises α-L-fucose residues and the blood group H antigen. In contrast to $sGP_{90-135}$, the antigen recognised by SWA20 bound slightly less of Triticum vulgaris and was unreactive with Concanavalin A. In a similar manner to $sGP_{90-135}$ there was an absence of binding of the lectin Ulex europaeus and succinylated Triticum vulgaris (succinylated Triticum vulgaris has no affinity to neuraminic acid, but recognises only N-acetyl-β-D-glucosaminyl residues). Assays were likewise performed to determine the effects of various enzymes on the antigen. In these assays, the enzyme neuraminidase (Clostridium perfringens) was employed, and also mixed glycosidases (a mixture of exoglycosidases from Charonia lampas containing mannosidase, glucosidase, galactosidase, fucosidase, xylosidase, acetylglucosaminidase and acetylgalactosaminidase), and assays with the proteases trypsin (N-tosyl-L-phenylalanylchloromethyl ketone) and chymotrypsin (tosyl-lysine chloromethyl ketone) were performed.

Similarly to $sGP_{90-135}$, the antigen $sGP_{100}$ was resistant to the mixed glycosidases and showed a parallel sensitivity to neuraminidase.

SW2 cells were also treated with periodate for 15 minutes at RT, and here, unlike $sGP_{90-135}$, the antigen of the invention was partially resistant.

These assays were repeated but with the prior incubation of the SW2 cell extracts with periodate and enzymes before separation on SDS-PAGE and development with antibody SWA20 (Figure 2). On this occasion, the treatment with periodate resulted in the disappearance of the bands at Mr 40,000 and Mr 100,000, but persistance of the Mr 180,000 band. Treatment with neuraminidase had a similar effect but in addition, new bands became visible at Mr 45,000. Treatment with the mixed glycosidases removed the Mr 40,000 bands and there was no effect seen with the enzymes trypsin and chymotrypsin. This is in contrast with similar treatments on $sGP_{90-135}$ wherein after treatment with periodate and neuraminidase all specific bands disappeared and treatment with chymotrypsin reduced the size of the middle range bands.

The reactivity of the antibodies LAM8 and SWA20 with the major blood group antigen $Le^a$ was examined by dot blot assays, in which small cell carcinoma extracts served as control. LAM8 is known to recognise a component in the saliva of $Le^a$ positive probands (Cancer Res. 47 [1987] 3766), but the monoclonal antibody SWA20 was shown to demonstrate no such reactivity (Figure 2).

Although there would appear to be similarities between the antigen $sGP_{90-135}$ and the antigen of the present invention, $sGP_{100}$, such as for example the apparent co-expression of these two antigens on the small cell carcinoma cell lines used in this investigation, the evidence for the sialylation of both antigens and the similarities in the molecular weights, there exist subtle differences between the two. The major differences between the two antigens are best demonstrated by the properties of the antibodies which identify them. Thus the differences are by the absence of reactivity of the monoclonal antibody SWA20, which identifies antigen $sGP_{100}$, with $Le^a$ positive saliva, the lack of binding competition to small cell carcinoma target cells between radiolabeled SWA20 and LAM8 (Table 1) and the lack of reactivity of the antibody SWA20 with immunoabsorbed $sGP_{90-135}$ (R.A. Stahel et al., Lung Cancer 4 [1988] 111-114; R. Waibel et al., Cancer Res. 48 [1988] 4318-4312). Between the antigens themselves, the differences are demonstrated by a relative resistance of $sGP_{100}$ to periodate and a persistance of the high molecular weight band of $sGP_{100}$ after treatment with one unit of periodate and neuraminidase. These findings clearly demonstrate that despite some similarities, the antigens recognised by the two antibodies are different from each other.

TABLE 1

| Competition radioimmunoassays with radiolabeled antibodies SWA20 and LAM8 on SW2 cells: | | |
|---|---|---|
| LABELED ANTIBODY | UNLABELED ANTIBODY | PERCENT BINDING* |
| SWA20 | None | 100 |
|  | SWA20 | 7 |
|  | LAM8 | 73 |
| LAM8 | None | 100 |
|  | SWA20 | 97 |
|  | LAM8 | 20 |

* expressed as percent binding without unlabeled antibody

The reason for co-expression of the antigens $sGP_{100}$ and $sGP_{90-135}$ and the lack of their expression on most normal cells remains to be determined. It could be speculated that small cell carcinoma cells expressing these antigens have a common alteration in the activity of enzymes responsible for post-translational sialylation of membrane proteins.

In addition to SWA20, further monoclonal antibodies have been produced having properties very similar to SWA20 and recognizing the same antigen. These are as indicated hereunder, together with their isotype:

| SEN3 | $IgG_1$ |
|---|---|
| SEN11 | $IgG_3$ |
| SEN12 | $IgG_{2b}$ |
| SEN31 | $IgG_1$ |

They are described in more detail in Example 6.

In using the monoclonal antibodies of the invention for the in vivo detection of antigen, the detectably labeled monoclonal antibody is given in a dose which is diagnostically effective. The term "diagnostically effective" means that the amount of detectably labeled monoclonal antibody is administered in sufficient quantity to enable detection of the site having the small cell carcinoma antigens for which the monoclonal antibodies are specific. The concentration of detectably labeled monoclonal antibody which is administered should be sufficient such that the binding to the tumour site is detectable compared to the background signal. Further, it is desirable that the detectably labeled monoclonal antibody be rapidly cleared from the circulatory system in order to give the best tumour-to-background signal ratio.

As a rule, the dosage of detectably labeled monoclonal antibody for diagnosis will vary depending on such factors as the age, sex and extent of disease of the individual. The dosage of monoclonal antibody can vary from about 1 mg to about 10 g, preferably from about 2 mg to about 1 g.

The monoclonal antibodies of the invention can also be used, alone or in combination with therapeutic agents, for immunotherapy of an animal. These agents can be coupled either directly or indirectly to the monoclonal antibodies of the invention. Examples of therapeutic agents which can be coupled to the monoclonal antibodies of the invention for immunotherapy are drugs, radioisotopes, immunomodulators, lectins or toxins.

For example, ricin is a toxic lectin which has been used immunotherapeutically and this is accomplished by binding the alpha-peptide chain of ricin, which is responsible for its toxicity, to the antibody molecule to enable site specific delivery of the toxic effect. Example 2 shows that the treatment of a small cell carcinoma cell line with an immunotoxin conjugate formed between an antibody of the invention, SWA20, and the A chain of ricin greatly reduces the incorporation of $^3$H leucine by the cells thus demonstrating the cytotoxic effect of such a conjugate.

In a similar manner toxins such as the diphtheria toxin fragment A produced by the micro-organism Corynebacterium diphtheriae may be linked to the antibody and used for site specific delivery of the toxin molecule. Such toxin fragments may be linked to the antibody using linker molecules or such techniques, or may otherwise be produced by a gene fusion and expression of the hybrid protein directly.

Other therapeutic agents which can be coupled to the monoclonal antibodies of the invention are known, or can readily be ascertained by those of ordinary skill in the art.

The dosage ranges for the administration of the monoclonal antibodies of the invention are those large enough to produce the desired effect in which the symptoms of the small cell carcinoma expressing the antigen sGP$_{100}$ are ameliorated. The dosage should not be so large as to cause adverse side effects, such as unwanted cross-reactions, anaphylactic reactions and the like. Generally, the dosage will vary with the age, condition, sex and the extent of the disease in the patient and can be determined by one of skill in the art. Dosage can vary from about 1 mg to about 10 g, preferably from about 2 mg to about 1 g. The antibodies can be administered parenterally by injection or by gradual perfusion over time. The monoclonal antibodies of the invention can be administered intravenously, intraperitoneally, intramuscularly, subcutaneously, intracavity or transdermally, alone or in combination with effector cells.

It will be appreciated that the isolation and characterization of the antigen with which for example SWA 20 is reactive, enables the possibility of virtually unlimited generation of additional monoclonal antibodies reactive with that antigen. This may be effected according to methods known in the art.

The antigen itself may also find application in the therapy of small cell carcinoma, e.g. in the production of appropriate vaccines.

The above disclosure generally describes the present invention. A more complete understanding can be obtained by reference to the following specific Examples which are provided herein for purposes of illustration only, and are not intended to limit the scope of the invention.


**EXAMPLE 1: PRODUCTION AND PURIFICATION OF ANTIBODY**


The procedure for the generation and selection of hybrid cell lines producing the monoclonal antibody of the invention has been reported (Cancer Res. 46: 2077 [1986] and Int. J. Cancer 35: 11 [1985]). Briefly, BALB-C mice were immunised with viable cells of the small cell carcinoma cell line SW2. Spleen cells were collected and fused with NS-1 myeloma cells to form the hybridoma. Selected antibodies were those which showed a ring pattern of activity with the cell line SW2 but not with leukaemia cells (CEM), adenocarcinoma of the lung (A549) or squamous cell carcinoma of the lung (HOTZ). Further screening for negative reactivity with bone marrow and screening for IgG isotype led to the selection of a hybridoma cell line which secreted a monoclonal antibody designated SWA20.

Antibody purification

The antibody SWA20 was purified by adsorbing a 30-55 % ammonium sulphate fraction of the culture supernatant to Protein A in binding buffer (1.5M glycine/NaOH, pH 8.9 and 3M NaCl). The bound immunoglobulin was desorbed with 100mM citrate buffer, pH 4.5, and dialysed against 10mM phosphate buffer, pH 6.8, with 0.01 mM $CaCl_2$. Antibody was then applied to a hydroxylapatite column (BioGel HPHT) and eluted with a linear gradient to 350 mM phosphate. The active fractions were pooled, dialysed against PBS, concentrated on a Speed Vac concentrator and stored at -70°C for further use.

The antibody LAM8 was purified according to the method of Parham (Methods in Enzymology 73 - [1981] 407). Briefly, this was accomplished by applying the 30-55 % ammonium sulphate fraction of the culture supernatant to a Superose 12 prep column (Pharmacia) in PBS. The peaks which were positive for IgM were pooled, precipitated by dialysis against 5 mM Tris, pH 7, and collected by centrifugation at 45,000 g for one hour at 4°C. LAM8 antibody was redissolved in PBS, sterile filtered and stored at 4°C for further use.

Purified antibody SWA20 was labeled by the Cloramine-T method to a specific activity of 500 $\mu$Ci/mg. Purified LAM8 antibody was labeled with a solid phase iodination system (Protag-125) to a specific activity of 54 $\mu$Ci/mg. With trichloroacetic acid uptake, 96 % of the activity was precipitable. The target uptake after labeling was determined and reached approximately 50 % for antibody SWA20 and 71 % for antibody LAM8.

## EXAMPLE 2: ANTIBODY-TOXIN CONJUGATE

An immunotoxin conjugate between the monoclonal antibody SWA20 and the A chain of ricin was formed by standard coupling techniques. Various dilutions of the resulting immunotoxin were incubated overnight with cells of the small cell carcinoma cell line NCI-h69, at a concentration of $10^5$ cells, in 96 well microplates, either with or without the addition of 50 nM of the ionophore HSA-monensin. $^3$H-leucine was added and incorporation was measured after 4 hours.

TABLE 2

| Immunotoxin | $IC_{50}$ |
|---|---|
| Ricin-A & HSA-monensin | $10^{-6}$ M |
| SWA20-ricin-A | $5 \times 10^{-9}$ M |
| SWA20-ricin-A & monensin | $5 \times 10^{-10}$ M |
| $IC_{50}$ = concentration causing 50 % inhibition | |

## EXAMPLE 3: IMMUNOLOGICAL DETECTION OF ANTIGEN IN CELL LINES

1. Immunofluorescence staining. The procedure has been reported, (Cancer Res. 46 [1986] 2077). Briefly, cells were aliquoted at 2 x $10^5$ per tube, incubated with 25 $\mu$l of supernatant for 60 minutes at 37°C, washed, and incubated with 50 $\mu$l of fluorescin-conjugated goat anti-mouse antibody.

2. Solid phase radioimmunoassays. For this procedure target cells were bound to 96 well plates. The wells were coated with poly-L-lysine and 5 x $10^5$ cells fixed with glutaraldehyde to each well. The plates were stored at 4°C with 1% BSA and 0.2 % sodium azide in PBS. Before use, the wells were incubated with wash medium (Tris buffered saline, 5 % non-fat dry milk and 1 % gelatine) for 30 minutes to prevent non-specific binding. To examine antibody binding on the panel of cell lines, supernatant at saturating concentration for binding on SW2 cells was used. After one hour's incubation, the plates were washed 4 times with PBS and 1 % BSA and $^{125}$I-goat anti-mouse IgG immunoglobulin (0.2 $\mu$Ci/50 $\mu$l) was added to each well. Each plate was again washed as before and the individual wells cut out and counted in a gamma counter for one minute. Experiments were done in triplicate. Typical counts for 100 % antibody binding on SW2 cells were around 20,000 cpm.

## TABLE 3

Binding of antibody SWA20 to small cell carcinoma cell lines as demonstrated by indirect immunofluorescence (IF) and solid phase radioimmunoassay (RIA) wherein the results are expressed as the percentage binding on the small cell carcinoma cell line SW2:

| Cell line | IF | RIA |
|---|---|---|
| SW2 | + | 100 |
| NC-1 - H69 | + | 90 |
| OH-1 | + | 88 |
| GEM-95 | + | 34 |
| OH-3 | 0 | 12 |
| ZL-2 | 0 | 8 |
| HC-1 - H-128 | 0 | 4 |
| GM222 | 0 | 0 |

## TABLE 4

Binding of antibody SWA20 to tumour cell lines other than small cell carcinoma as determined by indirect immunofluorescence (IF) and solid phase radioimmunoassay (RIA) wherein the results are expressed as the percentage binding on a small cell carcinoma cell line:

| CELL LINE | | IF | RIA |
|---|---|---|---|
| **Lung tumours:** | | | |
| Adenocarcinoma | A549 | 0 | 10 |
| Squamous cell carcinoma | Sk-Mes1 | 0 | 15 |
| | CaLu1 | 0 | 9 |
| **Extrapulmonary tumours:** | | | |
| Breast carcinoma | MCF-7 | 0 | 21 |
| | T470 | 0 | 14 |
| | ZR75-1 | 0 | 9 |
| | BT20 | 0 | 26 |
| Colon carcinoma | SW480 | 0 | 5 |
| Leukaemia | CEM | 0 | 0 |

## EXAMPLE 4: IMMUNOLOGICAL DETECTION OF ANTIGEN IN TISSUES

Immunoperoxidase staining of tissues for the detection of antigens may be effected in one of two manners:

1. Avidin-biotin method. Sections from parafin-embedded tissue blocks were cut at 5 $\mu$m, placed on gelatin covered glass slides, and incubated at 37°C for 16-18 hours. The sections were then deparaffinised in xylene, rehydrated in graded alcohols and washed in PBS. Subsequent incubations were performed at 37°C in a humidity chamber. Endogenous peroxidase activity was quenched with freshly prepared 1% $H_2O_2$ in methanol for 15 minutes. The sections were rinsed twice in PBS before incubation with normal goat (LAM8) or normal horse (SWA20) serum for 10 minutes to reduce non-specific background staining. Each section was exposed to 100 $\mu$l of antibody (undiluted culture supernatant) for 2 hours, thoroughly washed in PBS and then incubated with biotinylated goat anti-mouse IgM (LAM8) or biotinylated horse anti-mouse IgG (SWA20) for 10 minutes. The samples were again washed in PBS and treated with avidin-biotin conjugated horseradish peroxidase for 10 minutes. After washing in PBS the sections were exposed to freshly prepared peroxidase substrate (3-amino-9-ethylcarbazole AEC, $H_2O_2$ in acetate buffer, pH 5.2) for 15 minutes, washed in PBS and counterstained vith Mayers hemalaun. After washing in PBS and distilled water the sections were mounted in glycerol gelatin. Sections of a small cell carcinoma incubated with non-immune mouse serum served as negative control and sections of a small cell carcinoma staining positive with both

11

antibodies served as positive control. A positive reaction was indicated by a red cellullar deposit evaluated by light microscopy.

## TABLE 5

Expression of the sialoglycoprotein antigen $sGP_{100}$ in primary lung tumours and normal lung determined by immunoperoxidase staining with antibody SWA20:

| | Number of cases examined | Intensity of antigen expression | | | | |
|---|---|---|---|---|---|---|
| | | Number of cases (percent)[1] | | | | |
| | | ++++ | +++ | ++ | + | 0 |
| Small cell carcinoma | 152 | 32 (21) | 36 (24) | 12 (8) | 9 (6) | 63 (41) |
| Adenocarcinoma | 25 | 0 (0) | 0 (0) | 0 (0) | 0 (0) | 25 (100) |
| Large cell carcinoma | 10 | 0 (0) | 0 (0) | 0 (0) | 0 (0) | 10 (100) |
| Squamous cell carcinoma | 40 | 0 (0) | 1* (3) | 0 (0) | 1 (3) | 38 (95) |
| Mesothelioma | 10 | 0 (0) | 0 (0) | 0 (0) | 0 (0) | 10 (10) |
| Lung Carcinoid | 50 | 0 (0) | 1 (2) | 0 (0) | 5 (10) | 44 (88) |
| Normal lung bronchus and parenchyma | 25 | 0 (0) | 0 (0) | 1 (4) | 0 (0) | 24 (96) |

* biopsy, on subsequent resection classified as small cell carcinoma

[1] ++++ : > 50 % positive cells;   +++ : from 10 % to 50 %;
   ++ : < 10 %;                      + : rare positive cells
      0 : none

2. Tissues fixed in alcohol-zinc-formol solution were cut into 5 μm sections and mounted on glued slides. The sections were deparaffinised and treated with methanol peroxide and 2% swine serum as blocking reagent. The sections were covered with antibody at dilutions of up to 1:500 and incubated for one hour at RT. After washes in 0.05 M Tris buffer, pH 7.6, and peroxidase conjugated swine anti-rabbit immunoglobulin diluted 1:30 and peroxidase conjugated swine anti-rabbit immunoglobulin diluted 1:60 were added sequentially. The reaction was localised with 3,3'-diamino-benzidine tetramonohydrate (20 mg/10 ml in Tris buffer). Slides were counterstained with hematoxylin. Control studies included substitution with an irrelevant antibody (anti-leucocyte common antigen).

TABLE 6

| Immunoperoxidase staining of tumour tissues using antibody SWA20: | |
| --- | --- |
| Pulmonary tumours | |
| Small cell carcinoma | ¶¶φφφφ + + +00 |
| Adenocarcinoma | 000000 |
| Squamous cell carcinoma | 00000 |
| Large cell carcinoma | 0000 |
| Mesothelioma | 000000 |
| Carcinoid | 00000 |
| Extrapulmonary tumours | |
| Breast carcinoma | +000000000 |
| Adenocarcinoma of the stomach | 00000 |
| Colon carcinoma | 00000000 |
| Ovarian carcinoma | 000000 |
| Renal cell carcinoma | 000000 |
| Islet cell tumour | 0 |
| Melanoma | 0000 |
| Glioblastoma multiforme | 00 |
| Oligodendroglioma | 000 |
| Medulloblastoma | 000 |
| Neuroblastoma · | 0000 |
| Ganglioneuroma | 00 |
| Proportion of cells staining positive: | |
| ¶ > 50%  φ = 10-50%  + < 10%  0 = no staining | |

13

TABLE 7

| Immunoperoxidase staining of normal tissues using antibody SWA20: | | |
|---|---|---|
| Origin | Number examined | Reactivity |
| Lung bronchus | 10 | Rare positive cells* |
| Lung parenchyma | 10 | negative |
| Stomach | 1 | negative |
| Colon | 2 | positive, 10% of cells |
| Liver | 4 | negative |
| Pancreas, exocrine | 1 | negative |
| Pancreas, islets | 1 | rare positive cells |
| Kidney | 5 | negative |
| Prostate | 1 | negative |
| Spleen | 1 | negative |
| Lymph node | 1 | negative |
| Skin | 2 | negative |
| Nevus | 2 | negative |
| Anterior pituitary | 1 | negative |
| Brain | 3 | negative |
| Peripheral nerve | 4 | negative |

* less than 2% of cells positive

## EXAMPLE 5: ANALYSIS OF THE NATURE OF THE ANTIGEN

### 1. Antigens transferred from SDS to nitrocellulose.

Electrophoresis was accomplished on 10 % SDS-polyacrylamide gels under reducing conditions using the buffer system of O'Farrel. Aliquots of $5 \times 10^6$ cells were transferred electrophoretically onto 0.2 μm nitrocellulose according to the method of Otter (Anal. Biochem. 162 [1987] 370) in a two step procedure, beginning with the elution of low-molecular weight molecules at low current density (1 mA/cm²) for one hour, followed by prolonged electrotransfer (20 hours) at high current density in transfer buffer (49.6 mM Tris, 384 mM glycine, 20 % methanol (v/v)), and 0.01 % lithiumdodecylsulphate. After electrotransfer, the nitrocellulose sheets were air-dried and quenched by incubation (30 minutes) in Tris-buffered saline (TBS; 25 mM Tris, 0.5 M NaCl, pH 8) containing 5 % non-fat dried milk. The sheet was then incubated for 24 hours at 4° C with continuous rotation in LAM8 and SWA20 supernatant. After washing (3 times 10 minutes) with the quenching buffer and 0.05 % Tween 20 the sheets were incubated with affinity-purified peroxidase-conjugated goat anti-mouse IgM (μ chain specific), diluted 1:1000 in TBS and 20 % fetal calf serum, or goat anti-mouse IgG diluted 1:500, for one hour. After washing as before, peroxidase activity was demonstrated using 4-chloro-1-naphthol as substrate.

### 2. Immunoadsorption.

For immunoadsorption, cell extracts were incubated overnight with LAM8 antibody followed by adsorption on goat anti-mouse IgM agarose. After extensive washing the antigen was eluted with 9 M urea and applied to SDS-PAGE. After blotting individual strips were incubated with LAM8 (IgM), SWA4 (IgM antibody not competing with LAM8) and SWA20 (IgG) and developed as above. Results are shown in Figure 5.

### 3. Antigens on thin layer chromatograms.

14

Glycolipids were isolated from SW2 cells as follows. Cell pellets ($10^7$ cells) were washed twice with PBS and lyophilised. The lipids were extracted using chloroform and methanol (2:1 by volume) with three times 2 minutes sonication. The extract was filtered through a 0.22 μm filter and evaporated on a Speed-Vac concentrator. Crude glycolipids were chromatographed on high performance thin layer chromatography silica-plates in chloroform:methanol:0.25% KCl (5:4:1). After drying, the plates were either developed with ninhydrin spray reagent or immunostained as follows: the chromatograms were soaked for two hours at room temperature in PBS and 5 % non-fat dry milk to quench non-specific binding. The plates were incubated overnight in supernatant, washed two times 30 minutes as before and incubated with affinity-purified peroxidase-conjugated goat anti-mouse IgG, diluted 1:500 in PBS and 10 % horse serum for 2 hours. After washing as before peroxidase activity was demonstrated using 4-chloro-1-naphthol as substrate.

### 4. Extraction of antigen from the SW2 cell line.

For Western blot analysis, cells were washed once in PBS and solubilised in 50 mM octyl-beta-D-thioglucoside, 25 mM octylglucoside, 1 mM EDTA, 1 mM phenylmethyl sulfonyl fluoride, 0.1 mM leupeptine and 50 μg/ml pepstatin A in ice-cold PBS for 30 minutes. After centrifugation for one hour at 130,000 x g, the supernatant was precipitated stepwise with 25 %, 50 %, 75 % and 100 % ammonium sulphate, dialysed against PBS and concentrated on a Speed-Vac concentrator. The 50 - 75 % ammonium sulphate fraction was used for the Western blot analysis of the LAM8 and SWA20 antigens.

### 5. Periodate and enzyme and lectin treatment of antigens.

a) For periodate treatment of the antigens, aliquots of $10^7$ cells were incubated with 5 mM 10 mM 20 mM and 40 mM periodate at pH 7.0 at RT for 15 minutes. The reaction was stopped with ethylene glycol.

b) For competition radioimmunoassays with lectins, the wells were preincubated with 0.1 mg Ulex europaeus, Triticum vulgaris or Concanavalin A per well for 60 minutes.

c) For enzyme treatment the target cells were pre-incubated for four hours at 37° C with one unit of neuraminidase or either 1 mg or 10 mg of mixed glycosidases or one unit of chymotrypsin or one unit of trypsin. Glycosidases are a mixture of exoglycosidases from Charonia lampas containing 0.3 unit/10 mg mannosidase, 0.3 unit galactosidase, 0.3 unit fucosidase, 1.7 unit acetylglucosaminidase and 0.5 unit acetylgalactosaminidase.

## TABLE 8

Characterisation of the epitope recognised by antibody SWA20 using RIA on SW2 target cells pre-treated with periodate or enzymes or preincubated with lectins. The results, expressed as percent binding on a small cell carcinoma cell line are compared with LAM8:

|  |  | SWA20 | LAM8 |
|---|---|---|---|
| Periodate treatment | 5 mM | 103 | 64 |
|  | 10 mM | 89 | 54 |
|  | 20 mM | 75 | 34 |
|  | 40 mM | 68 | 27 |
| Enzyme treatment |  |  |  |
| Neuraminidase |  |  |  |
| 0.1 unit, 1 unit |  | 11 | 6 |
| mixed glycosidases | 0.1 mg | 105 | 97 |
|  | 1.0 mg | 98 | 95 |
|  | 10.0 mg | 65 | 72 |
| Lectin pre-incubation |  |  |  |
| Ulex europaeus |  | 88 | 85 |
| Triticum vulgaris |  | 26 | 4 |
| Triticum vulgaris (succinylated) |  | 98 | 100 |
| Concanavalin A |  | 98 | 57 |

## EXAMPLE 6: FURTHER ANTIBODIES

In a manner analogous to Example 1 the following further monoclonal antibodies have been obtained:

| SEN3 | (IgG$_1$) |
|---|---|
| SEN11 | (IgG$_3$) |
| SEN12 | (IgG$_{2b}$) |
| SEN31 | (IgG$_1$) |

having features very similar to SWA20 described above and recognizing the same antigen. This appears from the following:

a) They have identical cell line reactivity patterns on 19 small cell carcinoma cell lines, namely SW2, CORL47, CORL24, CORL88, GEM95, H60, H69, OH1, LX1, H128, OH3, ZL2, D.C571, H249, N417, H524, H446 and H526.

b) The sensitivity of antigen to enzymes is very similar:

TABLE 9

| Sensitivity of antigen to enzyme + periodate (%) | | | | | |
|---|---|---|---|---|---|
| Enzyme | Monoclonal antibody | | | | |
| | SEN3 | SEN11 | SEN12 | SEN31 | SWA20 |
| Trypsin (1 unit) | 121 | 124 | 87 | 93 | 112 |
| Chymotrypsin (1 unit) | 56 | 54 | 69 | 77 | 64 |
| Neuraminidase (0.2 units) | 9 | 18 | 13 | 9 | 20 |
| Mixed glycosidase | | | | | |
| (1 mg) | 116 | 114 | 131 | 117 | 113 |
| (10 mg) | 113 | 117 | 127 | 115 | 114 |
| Periodate | | | | | |
| (5 mM) | 8 | 20 | 52 | 18 | 80 |
| (50 mM) | 3 | 14 | 72 | 14 | 90 |

c) They exhibit a similar pattern on immunoblot.

d) In direct immunoassays they show direct competition for SW2 target cell binding:

TABLE 10

| | Marked monoclonal antibody | | | |
|---|---|---|---|---|
| | SEN11* | SEN12* | SEN31* | SWA20* |
| SEN3 | 49 | 15 | 43 | 22 |
| SEN11 | 14 | 7 | 21 | 4 |
| SEN12 | 43 | 17 | 57 | 25 |
| SEN31 | 7 | 7 | 9 | 4 |
| SWA20 | 33 | 14 | 47 | 9 |

* = % binding normalized to non-competed antibody

This is strongly indicative that the above monoclonal antibodies all recognize the same antigen.

**Claims**

1. A monoclonal antibody or fragment thereof characterised in that it is specific for a tumour-associated cell surface membrane-expressed antigen of the small cell carcinoma of the lung and shows substantially no non-specific reactivity.

2. An antibody or antibody fragment according to claim 1 characterised in that it identifies a cell surface membrane-expressed antigen of the small cell carcinoma of the lung, which antigen is a sialylated glycoprotein and demonstrates molecular weight bands of 40,000, 100,000 and 180,000 as illustrated by immunoblotting of a 50-75% ammonium sulphate fraction of a small cell carcinoma cell extract by electrophoretic transfer onto a nitrocellulose filter under sequential low and high current density.

3. An antibody or antibody fragment according to claim 1 which is of IgG isotype.

4. An antibody or antibody fragment according to claim 3 which is of IgG2a isotype.

5. An antibody or antibody fragment according to claim 1 produced by or derived from the hybridoma cell line ECACC 88030201.

6. An antibody according to claim 1 which is SWA20.

EP 0 323 802 A2

7. A tumour-associated antigen selectively expressed on the cell surface membrane of a proportion of small cell carcinoma of the lung characterised in that it is a sialylated glycoprotein and demonstrates molecular weight bands of 40 000 100,000 and 180,000 as illustrated by immunoblotting of a 50-75% ammonium sulphate fraction of a small cell carcinoma cell extract by electrophoretic transfer onto a nitrocellulose filter under sequential low and high current density.

8. A tumour-associated antigen selectively expressed on the cell surface membrane of a proportion of small cell carcinoma of the lung characterised in that it is a sialylated glycoprotein and is identified by an antibody or antibody fragment according to claim 1.

9. A hybridoma cell line capable of secreting a monoclonal antibody as defined in claim 1.

10. The hybridoma cell line according to claim 9 which is cell line ECACC 88030201.

11. A method of detecting an antigen according to claim 7 or 8 which comprises subjecting a source suspected of containing said antigen to a diagnostically effective amount of a detectably labeled antibody or antibody fragment and determining whether said antibody binds to said source.

12. A method of suppressing small cell carcinoma of the lung which comprises administering to a subject in need of such treatment a therapeutically effective amount of an antibody or antibody fragment according to claim 1.

13. The method of claim 12 wherein said antibody is produced by the hybridoma cell line ECACC 88030201.

14. A pharmaceutical composition comprising an amount of an antibody or antibody fragment according to claim 1 sufficient to suppress small cell carcinoma of the lung and a pharmaceutical carrier therefor.

15. An antibody or antibody fragment according to claim 1 for use in the suppression of small cell carcinoma of the lung.

**Claims for the following Contracting States: GR and ES**

1. A process for the preparation of a monoclonal antibody or fragment thereof that is specific for a tumour-associated cell surface membrane-expressed antigen of the small cell carcinoma of the lung and shows substantially no non-specific reactivity, comprising isolating antibodies from a corresponding hybridoma cell and if desired appropriately fragmenting the resultant antibodies.

2. A process according to claim 1 characterized in that the antibody or antibody fragment produced identifies a cell surface membrane-expressed antigen of the small cell carcinoma of the lung, which antigen is a sialylated glycoprotein and demonstrates molecular weight bands of 40,000, 100,000 and 180,000 as illustrated by immunoblotting of a 50-75% ammonium sulphate fraction of a small cell carcinoma cell extract by electrophoretic transfer onto a nitrocellulose filter under sequential low and high current density.

3. A process according to claim 1 characterized in that the antibody or antibody fragment produced is of IgG isotype.

4. A process according to claim 1 characterized in that the antibody or antibody fragment produced is of IgG2a isotype.

5. A process according to claim 1 characterized in that the antibody or antibody fragment produced is produced by or derived from the hybridoma cell line ECACC 88030201.

6. The process according to claim 1 wherein the antibody produced is SWA20.

7. A process for the production of a hybridoma cell line capable of secreting a monoclonal antibody as defined in claim 1 which comprises fusing an appropriate spleen cell with an appropriate myeloma cell and selecting accordingly.

8. A process according to claim 7 for the production of a hybridoma cell line which is cell line ECACC 88030201.

9. A method for the production of a pharmaceutical composition which comprises mixing an antibody or antibody fragment according to claim 1 with a pharmaceutically acceptable carrier or diluent.

18

# FIG. 1

EP 0 323 802 A2

FIG. 2

FIG. 3

FIG. 4

FIG. 5